Europäisches Patentamt

European Patent Office

Office européen des brevets

(11) Publication number: **0 435 556 A1**

# EUROPEAN PATENT APPLICATION

(21) Application number: 90313943.4

(22) Date of filing: 19.12.90

(51) Int. Cl.5: **A61K 7/48, A23L 1/29**

(30) Priority: 25.12.89 JP 337266/89

(43) Date of publication of application:
03.07.91 Bulletin 91/27

(84) Designated Contracting States:
AT BE CH DE DK ES FR GB GR IT LI LU NL SE

(71) Applicant: Asama Chemical Co., Ltd.
20-3 Nihonbashi-kodenmacho Chuo-ku
Tokyo(JP)

Applicant: Izumitani, Maremitsu
18-9, Tokura 3-chome
Kokubunji-shi, Tokyo(JP)

Applicant: MAMIE CORPORATION
16-10, Ohi 3-chome, Shinagawa-ku
Tokyo(JP)

(72) Inventor: Izumitani, Maremitsu
18-9, Tokura 3-chome
Kokubunji-shi, Tokyo(JP)
Inventor: Yajima, Mizuo
208, 9-16 Shirakawa, 4-chome
Koto-ku, Tokyo(JP)

(74) Representative: Taylor, Phillip Kenneth et al
W.P. THOMPSON & CO. Coopers Building
Church Street
Liverpool L1 3AB(GB)

(54) Tannin-containing composition.

(57) A beautifying composition comprising an effective amount of tannin(s) and a beautifying carrier such as a food or a drink, for example, coffee, chocolate, tea, gum, candy, sweets, cookie, cereal, wine, liquor, fruit juice, carbonated beverage and jam, or a cosmetic, especially a cosmetic material containing a vitamin(s).

# TANNIN-CONTAINING COMPOSITION

This invention relates to a beautifying composition containing tannin which is available as a food or a cosmetic having a high beautifying effect.

It has been a dearest wish of every woman to have a beautiful and white skin. Thus foods and cosmetics containing a beautifying agent have attracted great attention of consumers.

Although some of conventional foods of the aforesaid type contain ascorbic acid, this acid is unstable and liable to undergo decomposition or denaturation when exposed to, for example, heat, light, enzymes, metal ions or pH change, which makes it unsatisfactory.

Further, there are some drugs which aim at making the skin white and beautiful and treating pigmentation such as spots and freckles, for example, external skin preparations comprising SH compounds such as cysteine or glutathione, vitamin C, colloidal sulfur or hydroquinone. However these preparations have some disadvantages such that those comprising ascorbic acid are poor in stability; that those comprising colloidal sulfur cannot achieve a satisfactory beautifying effect; and that those comprising hydroquinone show some side effects, thus making all of them unsatisfactory.

The present inventors have found out that the intake of tannin as a food in an encapsulated or wafer-wrapped form, the administration of tannin as an ingredient of a nourishing or tonic agent or the use of a cosmetic containing tannin have a beautifying effect, thus achieving the present invention.

The invention provides a beautifying composition comprising an effective amount of tannin(s) and a beautifying carrier such as a food and a drink, for example, coffee, chocolate, tea, gum, candy, sweets, cookie, cereal, wine, liquor, fruit juice, carbonated beverage and jam, and then a cosmetic, especially a cosmetic material containing a vitamin(s).

The invention further provides a method for beautifying the skin of human being by administering tannin with a drink or a food and then a method for beautifying the skin of human being by applying tannin on the skin with a cosmetic.

The invention includes tannin added to a prior beautifying agent such vitamin C tablets or capsules.

The cosmetic to use in the invention may further include vitamins such as beta-carotin, vitamin C, a derivative from vitamin C, vitamin E, a derivative from vitamin E and vitamin A acid (retinoic acid); saccharide alcohol such as glycerin, xylitol and sorbitol; peptide such as casein peptide, collagen peptide and keratin peptide; or others for example including chitin, chitosan, elastin, hyaluronic acid, squalene, sodium chondroitin sulfate, nucleic acid, DNA, kojic acid (5-hydroxy-2-hydroxymethyl-gamma-pyrone), glycyrrhizin,
lethicin, sitosterol or glutathione.

Accordingly, a tannin-containing composition of the present invention is characterized by exerting a beautifying effect when taken in an amount of approximately 250 mg per day as a beautifying food in an encapsulated or wafer-wrapped form repeatedly for several months.

Another tannin-containing composition of the present invention is characterized by exerting a beautifying effect when added to, for example, a cosmetic which is to be repeatedly applied to the skin suffering from spots, freckles or dark skin.

The term "beautifying effect" as used herein means an effect whereby spots and freckles are removed and the skin is made beautiful, white and glossy.

In the present invention, tannin means an astringent substance commonly contained in, for example, root, stem, leaf, cortex and fruit of plants.

Typical examples of the tannin include persimmon tannin, chinese nutgall tannin, nutgall tannin, tea-leaf tannin, wattle tannin, quebracho tannin, dividivi tannin, myrobalan tannin and sumac tannin. Now each tannin will be briefly described.

Persimmon tannin:

Tannin contained in green astringent persimmons. A persimmon tannin extract is obtained by grinding green astringent persimmons, hermetically sealing the ground matter together with water and filtering it after several days. The obtained supernatant or a solution obtained by adding water to the cake followed by pressing affords a tannin extract. In general, the extract is stored in a hermetically sealed state for approximately 6 months prior to the use. A persimmon tannin powder is obtained by powdering the aforesaid persimmon tannin extract by, for example, spray drying, vacuum drying or lyophilizing.

Chinese nutgall tannin:

Tannin contained in a gall formed on a leaf of Rhus javanica (Anacardiaceae) by the sting of Kaburagia rhusiocola TAKAGI. A dry gall contains 50 to 70 % by weight of chinese nutgall tannin. It is available as a material for the formulation of, for example, astringent, remedy for hematemesis or remedy for catarrh.

Nutgall tannin:

Tannin contained in a gall formed on a young branch of a fagaceous tree by the stimulation caused by the oviposition of Cynips gallaetinctoria OLIV. A dry gall contains approximately 70 % by weight of nutgall tannin. It is one of crude drugs and available for nearly the same purposes as those described in the case of chinese nutgall tannin.

Tea-leaf tannin:

Tannin contained in a large amount in tea leaves. Dried fresh tea leaves contain 15 to 25 % by weight of tea-leaf tannin, though the content varies depending on the species of tea and the harvest time.

Wattle tannin:

Tannin contained in the cortex of various plants belonging to the genus Acacia. A dry cortex contains approximately 30 % by weight of wattle tannin.

Quebracho tannin:

Tannin extracted from the duramen of quebracho belonging to the family Rhus. A duramen contains approximately 20 % by weight of quebracho tannin.

Dividivi tannin:

Tannin contained in dividivi which is a leguminous tall tree. A dry dividivi fruit contains approximately 50 % by weight of dividivi tannin.

Myrobalan tannin:

Tannin contained in the fruit of a plant belonging to the family Combretaceae. A dry myrobalan fruit contains approximately 20 to 40 % by weight of myrobalan tannin while the sarcocarp contains 40 to 50 % by weight of the same.

Sumac tannin:

Tannin contained in the leaf of sumac or other plants belonging to the family of Rhus. Dry fresh leaves contain 13 to 26 % by weight of sumac tannin.

Each of these tannins may be obtained in the form of a tannin extract by extracting with water. Further, the obtained tannin extract may be powdered by, for example, spray drying vacuum drying or lyophilizing to thereby give a tannin powder.

The tannins as cited above are merely given by way of example and it is needless to say that the present invention is not restricted thereby.

When the tannin-containing composition of the present invention is to be taken as a food in, for example, an encapsulated form, it may contain, in addition to the above-mentioned tannin extract or tannin powder, monosaccharides such as lactose, starch, wheat flour, proteins and peptide and dietary fibers such as cellulose and polydextrose. Further, tannin may be added to other nourishing drugs such as vitamin preparations or tonics.

In summary, the composition of the present invention is one containing any of the aforementioned tannins and may be formulated into capsule, tablet, granules, biscuit, drink, nourishing foods, and refreshing drink as well as cream, cosmetic lotion, pack, milky lotion, soap and detergent, each of which may be produced in a conventional manner.

The content of tannin in the tannin-containing composition of the present invention may range from 0.01 to 20 % by weight, preferably from 0.1 to 10 % by weight. It may be determined depending on the dose of the food or cosmetic. In a standard case, tannin may be taken in a dose of 250 mg per day, which roughly corresponds to the amount of tannin contained in an astringent persimmon. The method for the addition of tannin may be appropriately selected depending on the production process or the form of each food or cosmetic product.

The detailed mechanism of the achievement of the beautifying effect of the tannin composition of the present invention is unelucidated as yet. It is assumed, however, that tannin would inhibit the activity of a pigment-forming anzyme such as tyrosinase and stimulate skin cells to thereby promote the decomposition of melanin pigment which has been formed, thus exerting an effect of preventing pigmentation and beautifying the skin, since spots and freckles are formed when tyrosine is converted into melanin by tyrosinase followed by pigmentation.

As described above in detail, the tannin-containing composition of the present invention is effective as a beautifying food or cosmetic. Thus this composition can satisfy the requirement of not only female but also male consumers in the treatment of spots, freckles and dark skin.

[Examples]

To further illustrate the present invention, and not by way of limitation, the following Examples will be given, wherein all parts are by weight.

Example 1: Capsule

Persimmon tannin was given 25 female subjects (aged from 25 to 50) suffering from spots, freckles or dark skin in a dose of one capsule (250 mg) per day for 3 months (from June to August) to thereby examine the effect of the present invention. Then each subject evaluated the skin condition items [(1) smoothness, (2) spots and freckles and (3) whiteness] by herself. Table 1 gives the results. As Table 1 indicates, the persimmon tannin had a beautifying effect.

## Table 1

| Extent of improvement | Remarkable | Moderate | Slight | No |
|---|---|---|---|---|
| (1) Smoothness | 2 | 3 | 5 | 15 |
| (2) Spots, freckles | 5 | 8 | 6 | 6 |
| (3) Whitening | 4 | 7 | 7 | 7 |

Example 2: Nourishing food

| | |
|---|---|
| vitamin B$_6$ | 0.05 part |
| vitamin B$_2$ | 0.01 " |
| chinese nutgall tannin | 0.45 " |
| royal jelly | 0.1 " |
| malic acid | 2.0 " |
| lactose | 25 " |
| granulated sugar | 30 " |
| glucose | the balance |
| in total | 100 |

The above components are mixed together to thereby give a nourishing food.

Example 3: Soft drink

| | |
|---|---|
| malic acid | 0.6 part |
| tartaric acid | 0.4 " |
| honey | 5.0 " |
| D-sorbitol (70 %) | 6.0 " |
| granulated sugar | 7.0 " |
| sodium alginate | 0.15 " |
| cinnamon oil | 0.003 " |
| tea-leaf tannin | 2.0 " |
| purified water | the balance |
| in total | 100 |

The above components were mixed together and uniformly dissolved. After filtering, a soft drink was obtained.

Example 4: Cosmetic lotion

| 1,3-butylene glycol | 3.0 parts |
| sorbitol solution (30 %) | 4.0 " |
| polyoxyethylene sorbitan monolaurate (20 E.O.) | 1.8 " |
| polyoxyethylene lauryl ether (20 E.O.) | 0.3 " |
| ethanol | 15 " |
| nutgall tannin | 3 " |
| methyl p-hydroxybenzoate | 0.1 " |
| water | the balance |
| in total | 100 |

The above components were mixed together and uniformly dissolved to thereby give a cosmetic lotion. Approximately 1 g/day of the obtained cosmetic lotion was applied to the whole face of each of 25 female subjects (aged 25 to 50) suffering from spots, freckles or dark skin for 3 months (from June to August) to thereby examine the effect of the present invention.

Then each subject evaluated the skin condition items [(1) smoothness, (2) spots and freckles and (3) whiteness] by herself. Table 2 gives the results. As Table 2 indicates, the persimmon tannin had a beautifying effect.

Table 2

| Extent of improvement | Remarkable | Moderate | Slight | No |
|---|---|---|---|---|
| (1) Smoothness | 10 | 8 | 7 | 0 |
| (2) Spots, freckles | 7 | 9 | 9 | 0 |
| (3) Whitenign | 12 | 8 | 5 | 0 |

Example 5: Pack

| talc | 28 | parts |
|---|---|---|
| red oxide | 26 | " |
| olive oil | 3 | " |
| polyoxyethylene sorbitan monolaurate (40 E.O.) | 0.8 | " |
| wattle tannin | 10 | " |
| sorbitol solution (70 %) | 6 | " |
| methyl p-hydroxybenzoate | 0.1 | " |
| perfume | 0.1 | " |
| kaolin | the balance | |
| in total | 100 | |

The above components were well mixed together to thereby give a powdery pack.

Example 6: Emollient cream

| squalane | 7.0 | parts |
|---|---|---|
| stearic acid | 3.0 | " |
| cetanol | 5.0 | " |
| glycerol tri(caprylate/caprate) | 8.0 | parts |
| polyoxyethylene cetyl ether (25 E.O.) | 2.5 | " |
| lipophilic glycerol monostearate | 2.0 | " |

The above components were mixed together and melted at approximately 80 °C. Then 0.15 part of methyl p-hydroxybenzoate, 4.0 parts of 1,3-butylene glycol, 4.3 parts of a sorbitol solution (70 %), 1.5 parts of quebracho tannin, and water (the balance to give a total amount of 100 parts), heated to approximately 60 °C, were added thereto. The obtained mixture was uniformly stirred and then 0.1 part of a perfume was added thereto. The obtained mixture was cooled under stirring to thereby give an emollient cream.

## Claims

1. A beautifying composition comprising an effective amount of tannin(s) and a beautifying carrier.

2. The composition as claimed in claim 1, characterised in that the beautifying carrier is a food or a drink.

3. The composition as claimed in claim 2, characterised in that the beautifying carrier is coffee, chocolate, tea, gum, candy, sweets, cookie, cereal, wine, liquor, fruit juice, carbonated beverage or jam

4.  The composition as claimed in claim 1, in which the beautifying carrier is a cosmetic.

5.  The composition as claimed in claim 4, in which the beautifying carrier is a cosmetic material containing a vitamin(s).

6.  A method for beautifying the skin of a human being by administering tannin with a drink or a food.

7.  A method for beautifying the skin of a human being by applying tannin on the skin with a cosmetic.

## European Patent Office

## PARTIAL EUROPEAN SEARCH REPORT

which under Rule 45 of the European Patent Convention
shall be considered, for the purposes of subsequent
proceedings, as the European search report

Application number

EP 90313943.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | PATENT ABSTRACTS OF JAPAN, unexamined applications, C field, vol. 8, no. 8, December 20, 1984 THE PATENT OFFICE JAPANESE GOVERNMENT page 72 C 257 * Kokai-Nò. 59-148 712 (KINGO) * | 1,2,4, 7 | A 61 K 7/48 A 23 L 1/29 |
| X | EP - A1 - 0 214 317 (SOCIETE DES PRODUITS NESTLE S.A.) * Claims 1-3,9,10; examples * | 1,2,3 | |
| X | DE - C - 449 454 (A. KÜNZEL) * Page 1, lines 20-50 * | 1,4,7 | |
| X | GB - A - 1 184 922 (LABOROIRES RENE RAMBAUD) * Claims 1-3; page 1, lines 14-37 * | 1,4,7 | |

TECHNICAL FIELDS SEARCHED (Int. Cl.5)

A 61 K 7/00
A 23 L 1/00
A 23 L 2/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-5,7

Claims searched incompletely: -

Claims not searched: 6, Article 52(4), method for treatment of the human or animal body by therapy

Reason for the limitation of the search

Place of search: VIENNA

Date of completion of the search: 19-02-1991

Examiner: IRMLER

EPO Form 1505.1 03 82

| | DOCUMENTS CONSIDERED TO BE RELEVANT | | CLASSIFICATION OF THE APPLICATION (Int. Cl.⁵ |
|---|---|---|---|
| Category | Citation of document with indication, where appropriate, of relevant passages | Relevant to claim | |
| X | PATENT ABSTRACTS OF JAPAN, unexamined applications, C field, vol. 10, no. 310, October 22, 1986 THE PATENT OFFICE JAPANESE GOVERNMENT page 145 C 379 * Kokai-No. 61-122 209 (OSAKA) * | 1,4,7 | |
| X | PATENT ABSTRACTS OF JAPAN, unexamined applications, C field, vol. 9, no. 241, September 27, 1985 THE PATENT OFFICE JAPANESE GOVERNMENT page 29 C 306 * Kokai-No. 60-98 962 (OSAKA) * | 1,2 | **TECHNICAL FIELDS SEARCHED (Int. Cl.⁵** |
| X | PATENT ABSTRACTS OF JAPAN, unexamined applications, C field, vol. 7, no. 118, May 21, 1983 THE PATENT OFFICE JAPANESE GOVERNMENT page 90 C 167 * Kokai-No. 58-38 209 (SUNSTAR) * | 1,4,7 | |
| X | PATENT ABSTRACTS OF JAPAN, unexamined applications, C field, vol. 12, no. 15, January 16, 1988 THE PATENT OFFICE JAPANESE GOVERNMENT page 95 C 469 * Kokai-No. 62-171 662 (NAGANO) * | 1,2,3 | |
| A | TASCHENBUCH DER MODERNEN PARFÜMERIE UND KOSMETIK, 1966, Wissenschaftliche Verlagsgesellschaft M.B.H., Stuttgart H. JANISTYN | 1,4,5 | |

European Patent Office

## DOCUMENTS CONSIDERED TO BE RELEVANT

| Category | Citation of document with indication, where appropriate, of relevant passages | Relevant to claim |
|---|---|---|
| | * Pages 340-346 * | |

CLASSIFICATION OF THE APPLICATION (Int. Cl.)5

TECHNICAL FIELDS SEARCHED (Int. Cl.)5

EPO Form 1505.3   06.78